# EUROPÄISCHE PATENTSCHRIFT

(11) **EP 0 708 080 B1**
(45) Veröffentlichungstag und Bekanntmachung des Hinweises auf die Patenterteilung: **18.11.1998**
(21) Anmeldenummer: 95113266.1
(22) Anmeldetag: 24.08.1995
(51) Int. Cl.: C07C 249/02, C07C 251/22, C07C 251/24, C07F 7/18

(54) **Verfahren zur Herstellung von N,N'-Disubstituierten p-Chinondiiminen und deren Verwendung**
Process for preparing N,N'-disubstituted p-quinone diimines and their use
Procédé pour la préparation de diimines de p-quinones N,N'-disubstitué et leur utilisation

(30) Priorität: 21.10.1994 DE 4437666
(43) Veröffentlichungstag der Anmeldung: 24.04.1996
(73) Patentinhaber: HÜLS AKTIENGESELLSCHAFT, 45764 Marl (DE)
(72) Erfinder: Haas, Margret, D-53639 Königswinter (DE); Bernhardt, Günther, Dr., D-53757 St. Augustin (DE)
(74) Vertreter: Dörries, Hans, Dr.

(56) Entgegenhaltungen:
- US-A- 2 118 826
- HOUBEN-WEYL 'Methoden der Organischen Chemie Band VII/3b' , GEORG THIEME VERLAG , STUTTGART * Seiten 267, 275 *

## Beschreibung

Die Erfindung betrifft ein Verfahren zur Herstellung von N,N'-disubstituierten p-Chinondiiminen der allgemeinen Formel I 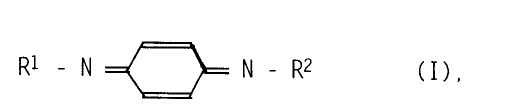 worin R¹ und R² gleich oder verschieden sind und eine Phenyl -Gruppe und/oder eine geradkettige und/oder verzweigte Alkyl-Gruppe mit 3 bis 8 C-Atomen und/oder eine Cyclohexyl-Gruppe bedeuten.

N,N'-disubstituierte p-Chinondiimine der allgemeinen Formel I sind keine Handelsprodukte, aber dennoch Verbindungen mit interessanten chemischen und physikalischen Eigenschaften.

Für die bekannten Verfahren zur Herstellung von N,N'-disubstituierten p-Chinondiiminen, nachfolgend auch kurz Diimine genannt, verwendet man als Ausgangsstoffe die korrespondierenden N,N'-disubstituierten p-Phenylendiamine, die unter Einwirkung von Oxidationsmitteln in die entsprechenden Diimine überführt werden. Nachfolgend werden die N,N'-disubstituierten p-Phenylendiamine auch als Diamine bezeichnet.

Die Verfahren zur Herstellung der Diimine liefern nach dem Stand der Technik entweder nur geringe Ausbeuten oder unreine Produkte, oder sie sind unwirtschaftlich, oder man verwendet überaus toxische Verbindungen bzw. diese entstehen während der Synthese.

So ist die Herstellung von N,N'-Diphenyl-p-chinondiimin aus dem korrespondierenden Diamin durch Oxidation mit Chromsäure in verdünnter, essigsaurer Lösung beschrieben (Chem. Ber. 46 (1913) S. 1853). Das gebildete Diimin fällt dabei in amorpher, schwer zu reinigender Form an und muß durch mehrmaliges Umkristallisieren gereinigt werden. Als Nebenprodukt entstehen relativ große Mengen einer Chrom-haltigen, wäßrigen, essigsauren Lösung, die nur aufwendig zu entsorgen ist.

Es ist weiter bekannt, N,N'-Diphenyl-p-chinondiimin durch Schütteln einer benzolischen Lösung von N,N'-Diphenyl-p-phenylendiamin mit einer wäßrigen Kaliumferricyanid-Lösung herzustellen (F. Feichtmayr u. F. Würstlin, Berichte der Bunsengesellschaft Bd. 67 (1963). S. 435). Da die Reaktionspartner in getrennten Phasen vorliegen, verläuft die Umsetzung sehr langsam und die Ausbeuten sind unbefriedigend.

Das gleiche Verfahren wird mit demselben Ergebnis auch für die Herstellung von N-Phenyl-N'-isopropyl-p-chinondiimin und N-Phenyl-N'-cyclohexyl-p-chinondiimin beschrieben (L. Kotulak et al., Collect. Czech. Chem. Commun. 48 (1983) 12, S. 3384-3395).

Auch die Verwendung von Silberoxid anstelle von Kaliumferricyanid als Oxidationsmittel wurde vorgeschlagen (L. Kotulak et al., Collect. Czech. Chem. Commun. 48 (1983) 12, S. 3384-3395). Silberoxid ist aber eine sehr teure und schlecht zu handhabende Chemikalie.

Die Verwendung von Brom als Oxidationsmittel führt zu teilweiser Kernbromierung der Chinondiimine (Houben/Weyl, Methoden der organischen Chemie Bd. 7/3b, 4. Aufl., S. 267).

Auch die Herstellung von N,N' -disubstituierten p-Chinondiiminen durch Luftoxidation der entsprechenden Diamine ist bekannt. So beschreibt E.v.Bandrowski, Monatshefte für Chemie, VIII. Band (1887), S. 478 die Herstellung von N,N'-Diphenyl-p-chinondiimin durch Oxidation von N,N'-Diphenyl-p-phenylendiamin mit Luftsauerstoff in einer siedenden alkoholischen Lösung, die zuvor mit Ätzkali versetzt war. Weiterhin wird N,N'-Diphenyl-p-phenylendiamin in Anwesenheit von hohen Überschüssen an Azoisobuttersäuredinitril (AIBN) durch Luftsauerstoff in ein Diimin umgewandelt (C. E. Boozer et al., Journ. Amer. Chem. Soc. 77 (1955) S. 3233). Nachteilig sind die Toxizität des eingesetzten AIBN, die Verwendung von Chlorbenzol als Lösemittel sowie die niedrigen Ausbeuten in diesem Verfahren.

In der US-PS 2 118 826 ist die Oxidation von N,N'-disubstituierten p-Arylendiaminen zu den korrespondierenden p-Chinondiiminen mit Luft in Gegenwart von festen Alkali- oder Erdalkalioxiden, -hydroxiden, -carbonaten oder -amiden beschrieben. Nachteilig ist hier die Feststoffreaktion, die hohen Reaktionstemperaturen von 130 bis 180 °C sowie die langen Reaktionszeiten von bis zu 8 Stunden, wobei eine erhebliche Zersetzung der gebildeten Diimine stattfinden kann, sind nicht von Vorteil.

Durch die zusätzliche Verwendung von Schwermetallsalzen als Katalysatoren können die Reaktionstemperaturen zwar abgesenkt werden, jedoch sind diese Salze nur schwer aus den erhaltenen N,N'-disubstituierten p-Chinondiiminen zu entfernen.

Der Erfindung liegt daher die Aufgabe zugrunde, ein einfaches und wirtschaftliches Verfahren zur Herstellung von N,N'-disubstituierten p-Chinondiiminen bereitzustellen.

Es wurde nun überraschenderweise gefunden, daß die Bildung des Diimins aus dem korrespondierenden N,N'-disubstiuierten Diamin besonders gut und mit hoher Produktausbeute in einem alkalisch/alkoholischen Milieu in Gegenwart von Halogenen als Oxidationsmitteln stattfindet. Unter einem korrespondierenden Diamin versteht man hier ein N,N'-disubstituiertes p-Phenylendiamin der allgemeinen Formel II 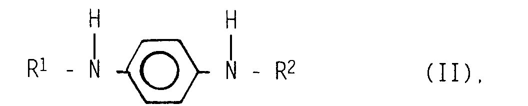 worin R¹ und R² dieselben organischen Restgruppen sind wie die in den N,N'-disubstituierten p-Chinondiiminen der allgemeinen Formel I. Als Oxidations-mittel erwiesen sich z. B. Chlor, Jod und insbesondere Brom als gut geeignet. Dies ist besonders überraschend, da eine zu erwartende Addition von Halogenen an das chinonide System unter den vorherrschenden Reaktionsbedingungen unterbleibt. Als besonders geeignetes alkalisch/alkoholisches Milieu erwiesen sich Alkalialkoholat-Lösungen. Das Produkt fällt in kristalliner Form an und kann z. B. durch Umkristallisieren in einfacher Weise nachgereinigt und vergleichsweise kostengünstig erhalten werden.

Gegenstand der vorliegenden Erfindung ist also ein Verfahren zur Herstellung von N,N'-disubstituierten p-Chinondiiminen der allgemeinen Formel I 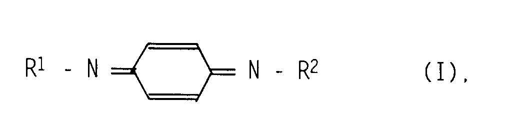 worin R¹ und R² gleich oder verschieden sind und eine Phenyl-Gruppe und/oder eine geradkettige und/oder verzweigte Alkyl-Gruppe mit 3 bis 8 C-Atomen und/oder eine Cyclohexyl-Gruppe bedeuten, das dadurch gekennzeichnet ist, daß die korrespondierenden N,N'-disubstituierten p-Phenylendiamine mit Halogenen als Oxidationsmittel in alkalisch/alkoholischer Lösung oxidiert werden.

Die N,N'-disubstituierten p-Chinondiiminen der allgemeinen Formel I eignen sich zur Stabilisierung von Methacryloxy- oder Acryloxy-Gruppen enthaltenden Organosilanen der allgemeinen Formel III 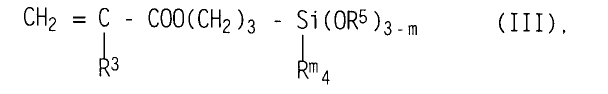 in der R³ ein Wasserstoffatom oder eine Methyl -Gruppe und R⁴ und R⁵ gleiche oder verschiedene Alkyl-Gruppen mit 1 bis 4 C-Atomen sind und m gleich 0 oder 1 oder 2 ist, während deren Herstellung, insbesondere während der destillativen Aufarbeitung im Sumpf und in der Dampfphase.

Als Edukte geeignete N,N'-disubstituierte Diimine sind z.B.:
N,N'-Diphenyl-p-chinondiimin,
N,N'-Di-(1-methylheptyl)-p-chinondiimin,
N,N'-Di-(1-ethyl-3-methylpentyl)-p-chinondiimin,
N,N'-Di-(1,4-dimethylpentyl)-p-chinondiimin,
N,N'-Di-sek.-butyl-p-chinondiimin,
N-Phenyl-N'-cyclohexyl-p-chinondiimin,
N-Phenyl-N'-isopropyl-chinondiimin.

Im erfindungsgemäßen Verfahren zur Herstellung von p-Chinondiiminen der allgemeinen Formel I wird als Oxidationsmittel vorzugsweise elementares Chlor oder Jod oder besonders vorzugsweise elementares Brom verwendet. Diese Halogene können in reiner Form, aber auch in einem geeigneten Lösemittel gelöst eingesetzt werden. Hierfür geeignete Lösemittel sind z. B. halogenierte Kohlenwasserstoffe. Bevorzugt wird jedoch das unverdünnte Halogen eingesetzt.

Geeigneterweise wird bei der Durchführung des erfindungsgemäßen Verfahrens ein Verhältnis der elektrochemischen Äquivalente von N,N 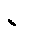 -disubstituierten p-Phenylendiaminen zu Halogen von 1:1 eingestellt. Das Halogen kann aber auch mit einem geringen Unterschuß eingesetzt werden, so daß es nach beendeter Oxidation restlos verbraucht ist.

Erfindungsgemäß erfolgt die Oxidation der Diamine der allgemeinen Formel II in einer alkalisch/alkoholischen Lösung als Reaktionsmedium.

Als Lösemittel können Alkohole, die auch mit Wasser gemischt werden können, verwendet werden. Insbesondere kommen aber reine Alkohole von technischer Qualität zum Einsatz. Als Alkohole werden vorzugsweise primäre oder sekundäre aliphatische Alkohole mit 1 bis 4 C-Atomen oder tertiäre aliphatische Alkohole mit 4 bis 8 C-Atomen, besonders vorzugsweise tert.-Butanol und ganz besonders vorzugsweise Methanol eingesetzt.

Als alkalischer Bestandteil der alkalisch/alkoholischen Lösung können Alkalihydroxide oder Alkalialkoholate eingesetzt werden. Bevorzugt aus der Reihe der Alkaliverbindungen sind hier die jeweiligen Natrium- und Kalium-Verbindungen, z.B. Natriumhydroxid, Kaliumhydroxid, Natriummethylat, Natriumethylat. Insbesondere sind die Alkalialkoholate hervorzuheben, wie z. B. Methylate und Ethylate. Besonders bevorzugt werden Kaliumalkoholate als alkalischer Bestandteil der alkalisch/alkoholischen Lösung eingesetzt, z. B. Kaliumethylat, Kalium-tert.-butylat, sowie ganz besonders bevorzugt Kaliummethylat. Geeigneterweise soll die Alkoholat-Konzentration der in einem der o.g. Alkohole gelösten Alkoholate 5 bis 32 Gew.-% betragen, sie kann aber auch davon abweichen. So sind z. B. technische, methanolische Kaliummethylat-Lösungen geeignet, diese können z.B. mit Methanol auf die gewünschte Konzentration verdünnt werden.

Für das erfindungsgemäße Verfahren kann das eingesetzte Diamin vollkommen oder auch nur teilweise in der verwendeten alkalisch/alkoholischen Lösung gelöst vorliegen.

Im erfindungsgemäßen Verfahren wird geeigneterweise ein Verhältnis von Gramm-Atom NH-aktivem Wasserstoff des N,N'-disubstituierten p-Phenylendiamins zu Gramm-Äquivalent des alkalischen Bestandteils von 1:1 eingestellt. Geringe Abweichungen im Verhältnis sind möglich, von 1:1 eingestellt. Geringe Abweichungen im Verhältnis sind möglich, jedoch werden damit keine verfahrenstechnischen Vorteile oder Ausbeuteverbesserungen erreicht.

Nach der durchgeführten Oxidation der Diamine zu Diiminen fällt das Oxidationsprodukt in der Regel in kristalliner Form an, während z. B. Kaliumbromid in Lösung verbleibt, was eine ökonomische Trennung von Zielprodukt und Nebenprodukt z.B. durch Filtration oder durch Zentrifugieren ermöglicht. Die so erhaltenen Diimine können durch gebräuchliche Aufarbeitungsmethoden, wie z.B. einer destillativen Aufarbeitung unter vermindertem Druck oder durch Säulenchromatografie in reiner Form dargestellt werden. Vorzugsweise erfolgt jedoch die Produktaufarbeitung durch einfaches Umkristallisieren, so werden N,N'-disubstituierte p-Chinondiimine mit hoher Reinheit erhalten.

Eine bevorzugte Variante des erfindungsgemäßen Verfahrens wird so durchgeführt, daß das N,N'-disubstituierte p-Phenylendiamin in einem Rührrektor unter Schutzgasatmosphäre, z. B. Stickstoff, ggf. unter leichtem Erwärmen in der alkalisch/alkoholischen Lösung gelöst, anschließend gekühlt und das Oxidationsmittel, elementar oder in gelöster Form, zudosiert wird. Die Oxidation der N,N'-disubstituierten p-Phenylendiamine wird vorzugsweise in einem Temperaturbereich zwischen 0 und 30°C, besonders vorzugsweise in einem Temperaturbereich zwischen 5 und 20°C durchgeführt.

Nach beendeter Reaktion sollte geeigneterweise das Oxidationsmittel verbraucht sein; eine Probe der Reaktionsmischung, die mit Wasser im Verhältnis 1:1 verdünnt wird, weist danach in der Regel einen pH-Wert >7 auf. Das kristalline Rohprodukt kann durch Filtration oder Zentrifugieren vom Reaktionsgemisch abgetrennt und durch Umkristallisieren in einem geeigneten Lösemittel oder durch Destillation unter vermindertem Druck nachgereinigt werden.

Es ist für die Verwendung der Diimine keine Vorbedingung, daß sie in analysenreiner Form vorliegen. Die erfindungsgemäß hergestellten N,N'-disubstituierten p-Chinondiimine ermöglichen es auch bei der Herstellung von Organosilanen der allgemeinen Formel III, insbesondere im PTC-Verfahren, in einfacher Weise die Polymerisation dieser Organosilane zu verhindern.

Die Erfindung wird durch die nachfolgenden Beispiele näher erläutert:

### Beispiele

### Beispiel 1

In einem Mehrhalskolben mit Rührer, Kühler, Gaseinleitungsrohr und Tropftrichter wurden 7,8 g (0,03 mol) N,N'-Diphenyl-p-phenylendiamin (Schmelzpunkt: 149-150°C), 150 g Methanol und 16,5 g (0,06 mol) einer 25,4 %igen Kaliummethylat-Lösung vorgelegt. Unter Rühren, Stickstoffzufuhr und Eiskühlung wurden 4,8 g (0,03 mol) Brom über 20 Minuten der Mischung zugetropft. Der ausgefallene rote Niederschlag wurde abfiltriert und der Filterrückstand mit wenig Methanol gewaschen (Schmelzpunkt: 173-175°C). Nach Umkristallisation aus Methanol/Aceton (1,14 : 1) wurden 5,9 g N,N'-Diphenyl-p-chinondiimin mit einem Schmelzpunkt von 181-183°C erhalten. Die Ausbeute lag bei 76,2 %.

### Beispiele 2 und 3

Beispiel 1 wurde dahingehend geändert, daß anstelle von N,N'-Diphenyl-p-phenylendiamin für das Beispiel 2 bzw. 3 jeweils das in Tabelle 1 aufgeführte N,N'-disubstituierte p-Phenylendiamin als Edukt verwendet wurde.

**Tabelle 1**

| Beispiel Nr. | Edukt | Produkt | Fp. [°C] | Ausbeute [%] |
|---|---|---|---|---|
| 2 | N-Phenyl-N'-cyclohexyl-p-phenylendiamin | N-Phenyl-N'-cyclohexyl-p-chinondiimin | 68-69 | 78,9 |
| 3 | N-Phenyl-N'-isopropyl-p-phenylendiamin | N-Phenyl-N'-isopropyl-p-chinondiimin | 42-43 | 80,1 |

## Patentansprüche

1. Verfahren zur Herstellung von N,N'-disubstituierten p-Chinondiiminen der allgemeinen Formel I 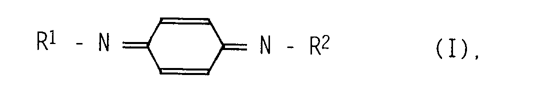 worin R¹ und R² gleich oder verschieden sind und eine Phenyl-Gruppe und/oder eine geradkettige und/oder verzweigte Alkyl-Gruppe mit 3 bis 8 C-Atomen und/oder eine Cyclohexyl-Gruppe bedeuten, dadurch gekennzeichnet, daß die korrespondierenden N,N'-disubstituierten p-Phenylendiamine mit Halogenen als Oxidationsmittel in alkalisch/alkoholischer Lösung oxidiert werden.

2. Verfahren nach Anspruch 1, dadurch gekennzeichnet, daß als Oxidationsmittel elementares Chlor oder Brom oder Jod verwendet wird.

3. Verfahren nach Anspruch 2, dadurch gekennzeichnet, daß als Oxidationsmittel elementares Brom verwendet wird.

4. Verfahren nach den Ansprüchen 1 bis 3, dadurch gekennzeichnet, daß ein Verhältnis der elektrochemischen Äquivalente von N,N'-disubstituierten p-Phenylendiaminen zu Halogen von 1:1 eingestellt wird.

5. Verfahren nach den Ansprüchen 1 bis 4, dadurch gekennzeichnet, daß als alkalischer Bestandteil der alkalisch/alkoholischen Lösung Alkalihydroxide oder Alkalialkoholate eingesetzt werden.

6. Verfahren nach Anspruch 5, dadurch gekennzeichnet, daß als alkalischer Bestandteil der alkalisch/alkoholischen Lösung Kaliumalkoholate eingesetzt werden.

7. Verfahren nach den Ansprüchen 1 bis 6, dadurch gekennzeichnet, daß ein Verhältnis von Gramm-Atom NH-aktivem Wasserstoff des N,N'disubstituierten p-Phenylendiamins zu Gramm-Äquivalent des alkalischen Bestandteils von 1 : 1 eingestellt wird.

8. Verfahren nach den Ansprüchen 1 bis 7, dadurch gekennzeichnet, daß als Alkohole primäre oder sekundäre aliphatische Alkohole mit 1 bis 4 C-Atomen oder tertiäre aliphatische Alkohole mit 4 bis 8 C-Atomen eingesetzt werden.

9. Verfahren nach Anspruch 8, dadurch gekennzeichnet, daß als Alkohol tert.-Butanol eingesetzt wird.

10. Verfahren nach Anspruch 8, dadurch gekennzeichnet, daß als Alkohol Methanol eingesetzt wird.

11. Verfahren nach den Ansprüchen 1 bis 10, dadurch gekennzeichnet, daß die Oxidation der N,N'-disubstituierten p-Phenylendiamine in einem Temperaturbereich zwischen 0 und 30 °C durchgeführt wird.

12. Verfahren nach Anspruch 11, dadurch gekennzeichnet, daß die Oxidation der N,N'-disubstituierten p-Phenylendiamine in einem Temperaturbereich zwischen 5 und 20 °C durchgeführt wird.

## Claims

1. A process for preparing N,N'-disubstituted p-quinonediimines of the general formula I 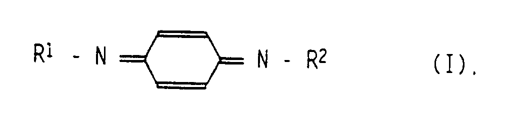 where R¹ and R² are identical or different and are each a phenyl group and/or a straight-chain and/or branched alkyl group having from 3 to 8 carbon atoms and/or a cyclohexyl group, characterized in that the corresponding N,N'-disubstituted p-phenylenediamines are oxidized in alkaline alcoholic solution using halogens as oxidizing agent.

2. A process according to claim 1, characterized in that elemental chlorine or bromine or iodine is used as oxidizing agent.

3. A process according to claim 2, characterized in that elemental bromine is used as oxidizing agent.

4. A process according to any of claims 1 to 3, characterized in that the ratio of electrochemical equivalents of N,N'-disubstituted p-phenylenediamines to halogen is set at 1:1.

5. A process according to any of claims 1 to 4, characterized in that alkali metal hydroxides or alkali metal alkoxides are used as alkaline constituent of the alkaline alcoholic solution.

6. A process according to claim 5, characterized in that potassium alkoxides are used as alkaline constituent of the alkaline alcoholic solution.

7. A process according to any of claims 1 to 6, characterized in that the ratio of gram atoms of NH-active hydrogen of the N,N'-disubstituted p-phenylenediamine to gram equivalents of the alkaline constituent is set at 1:1.

8. A process according to any of claims 1 to 7, characterized in that primary or secondary aliphatic alcohols having from 1 to 4 carbon atoms or tertiary aliphatic alcohols having from 4 to 8 carbon atoms are used as alcohols.

9. A process according to claim 8, characterized in that tert-butanol is used as alcohol.

10. A process according to claim 8, characterized in that methanol is used as alcohol.

11. A process according to any of claims 1 to 10, characterized in that the oxidation of the N,N'-disubstituted p-phenylenediamines is carried out in a temperature range from 0 to 30°C.

12. A process according to claim 11, characterized in that the oxidation of the N,N'-disubstituted p-phenylenediamines is carried out in a temperature range from 5 to 20°C.

## Revendications

1. Procédé de fabrication de p-quinonediimines N,N'-disubstituées de formule générale I 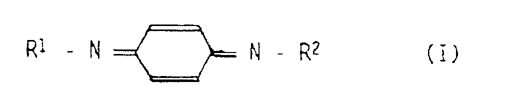 dans laquelle R¹ et R² sont identiques ou différents et représentent un groupe phényle et/ou un groupe alkyle à chaîne droite et/ou ramifiée, ayant de 3 à 8 atomes de carbone et/ou un groupe cyclohexyle
caractérisé en ce qu'
on oxyde les p-phénylènediamines N,N'-disubstituées correspondantes avec des halogènes comme oxydant dans une solution alcaline/alcoolique.

2. Procédé selon la revendication 1,
caractérisé en ce qu'
on utilise comme oxydant le chlore élémentaire, le brome ou l'iode.

3. Procédé selon la revendication 2,
caractérisé en ce qu'
on utilise comme oxydant le brome élémentaire.

4. Procédé selon les revendications 1 à 3,
caractérisé en ce qu'
on règle le rapport des équivalents électrochimiques des p-phénylènediamines N,N'-disubstituées aux halogènes à 1:1.

5. Procédé selon les revendications 1 à 4,
caractérisé en ce qu'
on utilise comme composant alcalin de la solution alcaline/alcoolique des hydroxydes alcalins ou des alcoolates de métaux alcalins.

6. Procédé selon la revendication 5,
caractérisé en ce qu'
on utilise comme composant alcalin de la solution alcaline/alcoolique des alcoolates de potassium.

7. Procédé selon les revendications 1 à 6,
caractérisé en ce qu'
on règle le rapport du nombre d'atomes-grammes d'hydrogène NH-actifs de la p-phénylènediamine N,N'-disubstituée au nombre d'équivalents-grammes du composant alcalin à 1:1.

8. Procédé selon les revendications 1 à 7,
caractérisé en ce qu'
on utilise comme alcools des alcools aliphatiques primaires ou secondaires ayant de 1 à 4 atomes de carbone ou des alcools aliphatiques tertiaires ayant de 4 à 8 atomes de carbone.

9. Procédé selon la revendication 8,
caractérisé en ce qu'
on utilise comme alcool le butanol tertiaire.

10. Procédé selon la revendication 8,
caractérisé en ce qu'
on utilise comme alcool le méthanol.

11. Procédé selon les revendications 1 à 10,
caractérisé en ce qu'
on procède à l'oxydation des p-phénylènediamines N,N'-disubstituées dans un intervalle de température compris entre 0 et 30°C.

12. Procédé selon la revendication 11,
caractérisé en ce qu'
on réalise l'oxydation des p-phénylènediamines N,N'-disubstituées dans un intervalle de température compris entre 5 et 20°C.
